(19) Europäisches Patentamt
European Patent Office
Office européen des brevets

(11) **EP 4 674 831 A1**

(12) **EUROPEAN PATENT APPLICATION**
published in accordance with Art. 153(4) EPC

(43) Date of publication:
**07.01.2026   Bulletin 2026/02**

(21) Application number: **24763493.4**

(22) Date of filing: **01.02.2024**

(51) International Patent Classification (IPC):
***C07C 17/383*** (2006.01)    ***C07C 19/08*** (2006.01)
***C07C 21/18*** (2006.01)

(52) Cooperative Patent Classification (CPC):
**C07C 17/383; C07C 19/08; C07C 21/18**

(86) International application number:
**PCT/JP2024/003332**

(87) International publication number:
**WO 2024/181009 (06.09.2024 Gazette 2024/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:   **28.02.2023   JP 2023030193**

(71) Applicant: **AGC INC.**
**Chiyoda-ku,**
**Tokyo 1008405 (JP)**

(72) Inventors:
 • **OTSUKA, Tetsuo**
  **Tokyo 100-8405 (JP)**
 • **MATSUNAMI, Asuka**
  **Tokyo 100-8405 (JP)**
 • **MITAKE, Akihito**
  **Tokyo 100-8405 (JP)**
 • **SHIOTA, Hidefumi**
  **Tokyo 100-8405 (JP)**
 • **KAMATSUKA, Tatsuya**
  **Tokyo 100-8405 (JP)**

(74) Representative: **Müller-Boré & Partner**
**Patentanwälte PartG mbB**
**Friedenheimer Brücke 21**
**80639 München (DE)**

(54) **METHOD FOR SEPARATING VINYLIDENE FLUORIDE AND TRIFLUOROMETHANE, COMPOSITION, AND METHOD FOR PRODUCING COMPOSITION**

(57)    A method of separating vinylidene fluoride and trifluoromethane, and the like are provided. The method includes: a step of mixing a mixture including vinylidene fluoride and trifluoromethane with a compound having a boiling point of from -60 to 0°C, to obtain a mixture for extraction; and a step of distilling the mixture for extraction, to independently obtain a distillate including vinylidene fluoride as a main component, and a bottom including a compound having a boiling point of from -60 to 0°C as a main component and further including trifluoromethane.

**EP 4 674 831 A1**

**Description**

Technical Field

The present disclosure relates to a method of separating vinylidene fluoride and trifluoromethane, a composition, and a method of producing a composition.

Background Art

Vinylidene fluoride is useful as a monomer for a fluorine resin.
For example, Patent Literature 1 describes a method of separating vinylidene fluoride and trifluoroethane, wherein the method includes: a step of adding a first extraction solvent which is at least one selected from the group consisting of alcohols having from 1 to 3 carbon atoms, ketones, esters, amides, ethers, sulfoxides, and nitriles to a first mixture including vinylidene fluoride and trifluoroethane, to obtain a second mixture; and an extractive distillation step of distilling the second mixture, to independently obtain a distillate including vinylidene fluoride as a main component, and a bottom including the first extraction solvent as a main component and including trifluoroethane.

Citation List

Patent Literature

Patent Literature 1: International Publication No. WO 2015/072305

SUMMARY OF INVENTION

Technical Problem

However, in Patent Literature 1, at least one selected from the group consisting of alcohols having from 1 to 3 carbon atoms, ketones, esters, amides, ethers, sulfoxides, and nitriles is used as an extraction solvent, and an energy required for distillation for collecting the extraction solvent has tended to be high.
One embodiment of the present invention addresses a task of providing: a method of separating vinylidene fluoride and trifluoromethane, wherein high-purity vinylidene fluoride can be obtained from a mixture including vinylidene fluoride and trifluoromethane, and an extraction solvent can be collected at a lower energy than in a conventional case; a composition associated with the separation method described above; and a method of producing a composition.

Solution to Problem

The present disclosure includes the following aspects.

<1>
A method of separating vinylidene fluoride and trifluoromethane, the method including:

a step of mixing a mixture including vinylidene fluoride and trifluoromethane with a compound having a boiling point of from -60 to 0°C, to obtain a mixture for extraction; and
a step of distilling the mixture for extraction, to independently obtain a distillate including vinylidene fluoride as a main component, and a bottom including a compound having a boiling point of from -60 to 0°C as a main component and further including trifluoromethane.

<2>
The method of separating vinylidene fluoride and trifluoromethane according to <1>, wherein an interaction distance $Ra^{R23}$ between trifluoromethane and the compound having a boiling point of from -60 to 0°C, determined from a Hansen solubility parameter value, is less than an interaction distance $Ra^{VdF}$ between vinylidene fluoride and the compound having a boiling point of from -60 to 0°C.
<3>
The method of separating vinylidene fluoride and trifluoromethane according to <2>, wherein a ratio of the interaction distance $Ra^{R23}$ to the interaction distance $Ra^{VdF}$ is 0.81 or less.
<4>
The method of separating vinylidene fluoride and trifluoromethane according to any one of <1> to <3>, wherein the compound having a boiling point of from -60 to 0°C has a boiling point of from -60 to -10°C.

<5>

The method of separating vinylidene fluoride and trifluoromethane according to any one of <1> to <4>, wherein the compound having a boiling point of from -60 to 0°C has a boiling point of from -60 to -30°C.

<6>

The method of separating vinylidene fluoride and trifluoromethane according to any one of <1> to <5>, wherein the compound having a boiling point of from -60 to 0°C is a hydrofluorocarbon.

<7>

The method of separating vinylidene fluoride and trifluoromethane according to any one of <1> to <5>, wherein the compound having a boiling point of from -60 to 0°C is at least one selected from a group consisting of difluoromethane, chlorofluoromethane, ammonia, isocyanic acid, ethyl azide, azidopropene, formyl fluoride, formaldehyde, and sulfur dioxide.

<8>

The method of separating vinylidene fluoride and trifluoromethane according to any one of <1> to <7>, wherein in the mixture for extraction, a ratio of a molar amount of the compound having a boiling point of from -60 to 0°C to a total molar amount of vinylidene fluoride and trifluoromethane is from 1 to 100.

<9>

The method of separating vinylidene fluoride and trifluoromethane according to any one of <1> to <8>, the method further including a step of distilling the bottom, to collect the compound having a boiling point of from -60 to 0°C.

<10>

The method of separating vinylidene fluoride and trifluoromethane according to <9>, wherein the collected compound having a boiling point of from -60 to 0°C is reused in the step of obtaining the mixture for extraction.

<11>

A composition including vinylidene fluoride, trifluoromethane, and a compound having a boiling point of from -60 to 0°C, wherein

a content of the vinylidene fluoride is 99% by mol or more with respect to a total amount of the composition.

<12>

A method of producing a composition, the method including:

a step of mixing a mixture including vinylidene fluoride and trifluoromethane with a compound having a boiling point of from -60 to 0°C, to obtain a mixture for extraction; and

a step of distilling the mixture for extraction, to obtain a composition including vinylidene fluoride as a main component.

Advantageous Effects of Invention

In accordance with one embodiment of the invention, a method of separating vinylidene fluoride and trifluoromethane, wherein high-purity vinylidene fluoride can be obtained from a mixture including vinylidene fluoride and trifluoromethane, and an extraction solvent can be collected at a lower energy than in a conventional case, a composition associated with the separation method described above, and a method of producing a composition are provided.

DESCRIPTION OF EMBODIMENTS

In the present disclosure, a numerical range expressed by "x to y" means a range including the values of x and y as the minimum and maximum values, respectively.

In a numerical range expressed in a stepwise manner in the present disclosure, the upper or lower limit value expressed in a certain numerical range may be replaced by the upper or lower limit value in another numerical range expressed in a stepwise manner. In a numerical range expressed in the present disclosure, the upper or lower limit value expressed in a certain numerical range may be replaced by values described in Examples.

In the present disclosure, a combination of two or more preferred aspects is a more preferred aspect.

In the present disclosure, in a case in which plural kinds of substances corresponding to each component exist, the amount of each component means, unless otherwise specified, the total amount of the plural kinds of substances.

In the present disclosure, "distillate" refers to a substance distilled from the column top side of a distillation column, and "bottom" refers to a substance distilled from the column bottom side of the distillation column.

In the present disclosure, "main component" means that the amounts of components other than the component are relatively smaller. The amount of "main component" is preferably 50% by mol or more, more preferably 60% by mol or more, still more preferably 70% by mol or more, and most preferably 80% by mol or more with respect to the total.

In the present disclosure, unless otherwise specified, the boiling point of a compound is a value at ordinary pressure, and the ordinary pressure is $1.013 \times 10^5$ Pa.

[Method of Separating VdF and R23]

A method of separating vinylidene fluoride (VdF) and trifluoromethane (R23) of the present disclosure includes: a step of mixing a mixture including VdF and R23 with a compound having a boiling point of from -60 to 0°C, to obtain a mixture for extraction; and a step of distilling the mixture for extraction, to independently obtain a distillate including VdF as a main component and a bottom including a compound having a boiling point of from -60 to 0°C as a main component and further including R23.

In accordance with the method of separating VdF and R23 of the present disclosure, high-purity VdF can be obtained from the mixture including VdF and R23, an extraction solvent can be collected from a mixture of R23 and the extraction solvent, obtained by separation of VdF, at a lower energy than in a conventional case, and separability between R23 and the extraction solvent is also excellent.

VdF is useful as a monomer for a fluorine resin. However, for example, in a case in which a polymerization reaction is performed using a composition including VdF and R23 as an impurity, R23 that is not consumed in the polymerization reaction in the later period of a polymerization step accumulates in the interior of a reactor, whereby the polymerization reaction is inhibited. As a result, the rate of the polymerization reaction is decreased, or the polymerization reaction is stopped, whereby a polymer of interest is prone to be prevented from being obtained. Therefore, it is desirable to separate VdF and R23, to obtain high-purity VdF. However, VdF has a boiling point of -83°C, and R23 has a boiling point of -82°C. VdF and R23 have the similar boiling points, and a mixture of VdF and R23 has an azeotropic composition. Therefore, it is difficult to separate and purify VdF and R23 by usual distillation.

Thus, Patent Literature 1 describes that extractive distillation is performed using an extraction solvent which is at least one selected from the group consisting of alcohols having from 1 to 3 carbon atoms, ketones, esters, amides, ethers, sulfoxides, and nitriles. The extraction solvent described in Patent Literature 1 has a high boiling point of from 40°C to 250°C. The boiling point of the extraction solvent is high. Therefore, distillation for collecting the extraction solvent included in a bottom after the extractive distillation has tended to require a large amount of energy.

The present inventors found that using a compound having a boiling point of from -60 to 0°C as an extraction solvent enables the extraction solvent to be collected at a lower energy than in a conventional case.

Each step in the method of separating VdF and R23 of the present disclosure is described below.

<Mixing Step>

The method of separating VdF and R23 of the present disclosure includes a step of mixing a mixture including VdF and R23 with a compound having a boiling point of from -60 to 0°C, to obtain a mixture for extraction (hereinafter also referred to as "mixing step").

(Mixture Including VdF and R23)

The mixture including VdF and R23 may include another component except VdF and R23, and the smaller content of the other component is preferred from the viewpoint of efficiently obtaining high-purity VdF. Examples of such another component include fluoroolefins except VdF, and hydrofluorocarbons except R23.

The total content of VdF and R23 with respect to the total amount of mixture including VdF and R23 is, for example, 50% by mol or more, and may be 80 mol or more, may be 90 mol or more, may be 99% by mol or more, and may be 100% by mol.

In the mixture including VdF and R23, the molar ratio between VdF and R23 is not particularly limited. From the viewpoint of production efficiency, the ratio (that is, molar ratio) of the molar amount of R23 to the molar amount of VdF is preferably from 0.01 to 1, more preferably from 0.01 to 0.5, and still more preferably from 0.01 to 0.1.

As the mixture including VdF and R23, for example, a reaction product containing VdF, obtained by allowing various raw materials to react with each other, can be used for the purpose of producing VdF.

For example, the reaction product containing VdF can be obtained by dehydrochlorination reaction using chlorodifluoroethane as a raw material. The reaction product containing VdF can be obtained by dehydrochlorination reaction using chlorodifluoromethane and chloromethane as raw materials.

(Compound Having Boiling Point of from -60 to 0°C)

The compound having a boiling point of from -60 to 0°C (hereinafter also referred to as "compound C") is regarded as an extraction solvent. The number of kinds of such compounds C may be one, or two or more.

The boiling point of the compound C is -60°C or more. Therefore, the difference between the boiling point of the compound C and the boiling point of R23 is large, and R23 is easily separated. The boiling point of the compound C is 0°C or less. Therefore, in a case in which the bottom is distilled to collect the compound C, the compound C can be collected at a lower energy than in a conventional case.

The compound C preferably has a boiling point of from -60 to -10°C, more preferably from -60 to -30°C, from the viewpoint of decreasing the temperature of the column bottom of the distillation column in the case of collecting the compound C by distillation and decreasing the amount of heat required for heating.

Specifically, the compound C is preferably a hydrofluorocarbon (HFC). Examples of such an HFC include difluoromethane (HFC-32). Such an HFC is preferred in view of having low reactivity with another compound and being prevented from self-decomposing. Such an HFC is preferred in view of being able to inhibit the degradation of a manufacturing apparatus due to a low redox property.

The compound C is preferably at least one selected from the group consisting of difluoromethane (HFC-32), chlorofluoromethane (HCFC-31), ammonia, isocyanic acid, ethyl azide, azidopropene, formyl fluoride, formaldehyde, and sulfur dioxide.

Especially, the compound C is preferably HFC-32 from the viewpoint of separability.

From the viewpoint of separability, an interaction distance $Ra^{R23}$ between R23 and the compound C, determined from a Hansen solubility parameter value, is preferably less than an interaction distance $Ra^{VdF}$ between VdF and the compound C. The ratio ($Ra^{R23}/Ra^{VdF}$) of the interaction distance $Ra^{R23}$ to the interaction distance $Ra^{VdF}$ is preferably 0.81 or less, more preferably 0.79 or less, and still more preferably 0.64 or less from the viewpoint of separating VdF and R23 with high efficiency.

The interaction distance $Ra^{VdF}$ and the interaction distance $Ra^{R23}$ are represented by the following Formulas (1) and (2), respectively.

$$\text{Formula (1): } Ra^{VdF} = [4 \times (\delta D^{VdF} - \delta D^{C})^2 + (\delta P^{VdF} - \delta P^{C})^2 + (\delta H^{VdF} - \delta H^{C})^2]^{0.5}$$

$$\text{Formula (2): } Ra^{R23} = [4 \times (\delta D^{R23} - \delta D^{C})^2 + (\delta P^{R23} - \delta P^{C})^2 + (\delta H^{R23} - \delta H^{C})^2]^{0.5}$$

In Formulas (1) and (2), $\delta D^{VdF}$, $\delta P^{VdF}$, and $\delta H^{VdF}$ represent a dispersion term, a polar term, and a hydrogen bonding term in the Hansen solubility parameter of VdF, respectively, $\delta D^{R23}$, $\delta P^{R23}$, and $\delta H^{R23}$ represent a dispersion term, a polar term, and a hydrogen bonding term in the Hansen solubility parameter of R23, respectively, $\delta D^{C}$, $\delta P^{C}$, and $\delta H^{C}$ represent a dispersion term, a polar term, and a hydrogen bonding term in the Hansen solubility parameter of the boiling point compound C, respectively, and each unit is $(MPa)^{1/2}$.

The dispersion term, the polar term, and the hydrogen bonding term in the Hansen solubility parameter of each compound described above are literature values or values estimated from chemical structure of the compound by computer software (Hansen Solubility Parameters in Practice (HSPiP) version 4). The Hansen solubility parameter of the mixture including two or more compounds is calculated as the vectorial sum of a value obtained by multiplying the Hansen solubility parameter of each compound by the volume ratio of each compound to the whole mixture.

In the mixture for extraction, the ratio of the molar amount of compound C to the total molar amount of VdF and R23 (that is, "compound C/(VdF + R23)") is preferably from 1 to 100, and more preferably from 1 to 50 from the viewpoint of separating VdF and R23 with high efficiency in the step of obtaining the mixture for extraction.

For example, as described later, "compound C/(VdF + R23)" represents the molar ratio of the supply amount of compound C supplied into the extraction column to the total supply amount of VdF and R23 supplied into the extraction column in the case of supplying the mixture including VdF and R23 into the extraction column and further supplying the compound C into the extraction column.

A method of mixing the mixture including VdF and R23 with the compound C is not particularly limited. The compound C may be added to the mixture including VdF and R23, or the mixture including VdF and R23 may be added to the compound C.

Timing at which the mixture for extraction is obtained is not particularly limited as long as the timing is prior to distillation of the mixture for extraction.

From the viewpoint of efficiency in the distillation of the mixture for extraction, described later, it is preferable that the compound C is supplied into the extraction column into which the mixture including VdF and R23 has been supplied, the mixture for extraction is prepared in the extraction column, and the distillation is performed at the end of the preparation.

In the mixture for extraction, examples of other components except VdF, R23, and the compound C include other components that may be included in the mixture including VdF and R23 described above.

<Extractive Distillation Step>

The method of separating VdF and R23 of the present disclosure includes a step of distilling the mixture for extraction, to independently obtain a distillate including VdF as a main component, and a bottom including a compound having a boiling point of from -60 to 0°C as a main component and further including trifluoromethane (hereinafter also referred to as "extraction separation step").

An extractive distillation step can be carried out using a distillation apparatus which is commonly used, for example, a distillation column such as a plate column, a packed column, or the like. Various conditions in the extractive distillation step, for example, an operation temperature, an operation pressure, a reflux ratio, the total number of plates of a distillation column, the position of a loading plate, the position of an extraction solvent supply plate, and the like are not particularly limited, and can be selected, if appropriate, to achieve separation of interest. In a case in which a distillation column which is a plate column is used in the extractive distillation step, examples of the number of plates of the distillation column include from 1 to 100, and, from the viewpoint of obtaining high-purity VdF, the number is preferably 30 or more, and more preferably 50 or more. Each of VdF and R23 has a low boiling point. Therefore, it is preferable to perform extractive distillation under an increased pressure, and, for example, a pressure of from 0 to 5 MPaG (gauge pressure) is preferred.

The temperatures of the column top and column bottom of the distillation column depend on an operation pressure and the compositions of the distillate and the bottom. In consideration of the temperatures of a condenser and a reheater disposed in the column top and the column bottom, it is preferable that the temperature of the column top is set at from -60 to 100°C, and the temperature of the column bottom is set at from -10 to 300°C in order to economically perform distillation operation. The extractive distillation may be batch-type or continuous-type, and a semi-continuous type extractive distillation in which a distillate and a bottom are intermittently extracted and intermittently loaded can also be optionally performed. However, it is preferable to continuously supply the extraction solvent into the distillation apparatus.

The compound C has an affinity for R23. Accordingly, the distillate including VdF as a main component is obtained from the column top side of the extraction column by extractive distillation of the mixture for extraction, including VdF, R23, and the compound C. The composition of the distillate is not limited as long as the distillate includes VdF as a main component. The mole fraction of VdF in the distillate is preferably 90% by mol or more, and more preferably 99% by mol or more. The mole fraction of R23 in the distillate is preferably not more than 1/10, more preferably not more than 1/100, of the mole fraction of R23 in the mixture for extraction. The mole fraction of R23 in the distillate is preferably 10% by mol or less, and more preferably 1% by mol or less.

The bottom including the compound C as a main component and further including R23 is obtained from the column bottom side of the extraction column. The composition of the bottom is not limited as long as the bottom includes the compound C as a main component. However, the mole fraction of the compound C in the bottom is preferably 30% by mol or more, and more preferably 50% by mol or more.

<Distillation Step>

The method of separating VdF and R23 of the present disclosure preferably further includes a step of distilling the bottom, to collect the compound C (hereinafter also referred to as "distillation step").

The distillation step can be carried out using a distillation apparatus similar to the distillation apparatus in the extractive distillation step described above. Various conditions in the distillation step, for example, an operation temperature, an operation pressure, a reflux ratio, the total number of plates of a distillation column, the position of a loading plate, and the like are not particularly limited, and can be selected, if appropriate, to achieve separation of interest.

The distillation step allows the compound C, and VdF and R23 to be separated, enabling the compound C to be collected.

In the distillation step, the bottom including the compound C is obtained from the column bottom side of the distillation column. The collected compound C is preferably reused in the extractive distillation step described above. The method of separating VdF and R23 of the present disclosure is excellent in separability between R23 and the compound C due to a large difference between the boiling points of R23 and the compound C. Therefore, the high-purity compound C can be collected, and is suitable for reuse.

[Composition]

A composition of one embodiment of the present disclosure is a composition including VdF, R23, and a compound C, and the content of VdF is 99% by mol or more, preferably 99.5% by mol or more, and more preferably 99.9% by mol or more with respect to the total amount of the composition.

In the composition of one embodiment of the present disclosure, R23 and the compound C are impurities, the smaller contents thereof are preferred, and it is desirable that R23 and the compound C are not substantially included. However, the compound C may be included from the viewpoint of the simplification of the production step.

The content of R23 is preferably 1% by mol or less, more preferably 0.5% by mol or less, and still more preferably 0.1% by mol or less with respect to the total amount of the composition. The content of R23 is preferably 1 ppm by mol or more, more preferably 5 ppm by mol or more, and still more preferably 10 ppm by mol or more with respect to the total amount of the composition.

The content of the compound C is preferably 1% by mol or less, more preferably 0.5% by mol or less, and still more preferably 0.1% by mol or less with respect to the total amount of the composition. The content of R23 is preferably 1 ppm by mol or more, more preferably 5 ppm by mol or more, and still more preferably 10 ppm by mol or more with respect to the total amount of the composition.

The composition of one embodiment of the present disclosure may include another component except VdF, R23, and the compound. Examples of such another component include components similar to the other components that may be included in the mixture including VdF and R23 described above.

The composition which includes VdF, R23, and the compound C, and in which the content of VdF is 99% by mass or more with respect to the total amount of the composition is obtained by, for example, separating the mixture including VdF and R23 by the separation method described above.

[Method of Producing Composition]

A method of producing a composition of one embodiment of the present disclosure includes: a step of mixing a mixture including VdF and R23 with a compound C, to obtain a mixture for extraction; and a step of distilling a mixture for extraction, to obtain a composition including VdF as a main component.

The step of obtaining the mixture for extraction is similar to the step of obtaining the mixture for extraction in the separation method of one embodiment of the present disclosure.

The step of distilling the mixture for extraction, to obtain the composition including vinylidene fluoride as a main component, is similar to the step of distilling the mixture for extraction, to obtain the distillate including vinylidene fluoride as a main component in the separation method of one embodiment of the present disclosure.

The method of producing a composition of one embodiment of the present disclosure may include: a step of mixing a mixture including VdF and R23 with a compound C, to obtain a mixture for extraction; and a step of distilling the mixture for extraction, to independently obtain a composition including VdF as a main component, and a composition including the compound C as a main component and further including R23.

Examples

The present disclosure is specifically described in detail below with reference to Examples. However, the present disclosure is not limited to these Examples.

HFC-32 (hereinafter referred to as "R32") was used as an extraction solvent (compound C).

Interaction distance $Ra^{R23}$ between R23 and R32: 3.6
Interaction distance $Ra^{VdF}$ between VdF and R32: 6.8

<Gas-Liquid Equilibrium Test (VdF and R23)>

A gas-liquid equilibrium test was conducted using VdF and R23.

VdF and R23 were put in a 75-mL autoclave with a pressure gauge, and gradually heated by an external heater so as to achieve a predetermined pressure. In a case in which the pressure reached the predetermined value, the pressure was then kept during a certain period, to stabilize the composition in the autoclave. Then, the samples of measurement specimens were collected from a gas phase and a liquid phase, respectively, and analyzed by gas chromatography, to measure the molar ratios of VdF and R23.

A relative volatility was calculated using the concentration of VdF in the gas phase (VdF gas phase concentration), the concentration of R23 in the gas phase (R23 gas phase concentration), the concentration of VdF in the liquid phase (VdF liquid phase concentration), and the concentration of R23 in the liquid phase (R23 liquid phase concentration).

Relative volatility $\alpha$ = {(VdF gas phase concentration/R23 gas phase concentration)}/{(VdF liquid phase concentration/R23 liquid phase concentration)}

The measurement results and the calculation results are set forth in Table 1.

<Gas-Liquid Equilibrium Test (VdF, R23, and R32)>

A gas-liquid equilibrium test was conducted using VdF, R23, and R32.

VdF, R23, and R32 were put in a 75-mL autoclave with a pressure gauge, and gradually heated by an external heater so as to achieve a predetermined pressure. In a case in which the pressure reached the predetermined value, the pressure was then kept during a certain period, to stabilize the composition in the autoclave. Then, the samples of measurement specimens were collected from a gas phase and a liquid phase, respectively, and analyzed by gas chromatography, to measure the molar ratios of VdF, R23, and R32. A relative volatility was calculated.

The measurement results and the calculation results are set forth in Table 2.

[Table 1]

| Gas phase | | Liquid phase | | Pressure [MPa-G] | Temperature [°C] | Relative volatility |
|---|---|---|---|---|---|---|
| VdF [% by mol] | R23 [% by mol] | VdF [% by mol] | R23 [% by mol] | | | |
| 9.2 | 90.8 | 8.4 | 91.6 | 1.94 | -8.5 | 1.105 |
| 27.6 | 72.4 | 26.8 | 73.2 | 2.13 | -7.2 | 1.041 |
| 42.3 | 57.7 | 43.3 | 56.7 | 1.93 | -9.6 | 0.960 |
| 68.5 | 31.5 | 71.8 | 28.2 | 1.81 | -9.2 | 0.854 |
| 87.1 | 12.9 | 89.3 | 10.7 | 1.7 | -10.3 | 0.809 |

[Table 2]

| Gas phase | | | Liquid phase | | | Pressure [MPa-G] | Temperature [°C] | Relative volatility |
|---|---|---|---|---|---|---|---|---|
| VdF [% by mol] | R23 [% by mol] | R32 [% by mol] | VdF [% by mol] | R23 [% by mol] | R32 [% by mol] | | | |
| 16.9 | 1.4 | 81.7 | 5.1 | 0.7 | 94.2 | 0.68 | -10.6 | 1.657 |

Based on Table 1, it was found that in a case in which the gas phase concentration of VdF was in a range of from 9.2% by mol to 27.6% by mol, the relative volatility was more than 1, and a large amount of R23 existed in the liquid phase. It was found that in a case in which the gas phase concentration of VdF was in a range of from 42.3% by mol to 87.1% by mol, the relative volatility was less than 1, and the mixture of VdF and R23 was an azeotropic mixture.

As set forth in Table 2, the addition of R32 caused the relative volatility of VdF and R23 to be 1.657. The concentration of VdF with respect to the total amount of VdF and R23 was more than 80% by mol, and the relative volatility was less than 1 (Table 1) in the case of the two components of VdF and R23. It was found that the addition of R32 caused the relative volatility to be more than 1, and R23 to be extracted into the liquid phase.

The results of the gas-liquid equilibrium test described above were reflected, and the simulation of extractive distillation and the simulation of distillation were performed using a process simulator Chemcad. In the simulations, distillation columns of which the number of plates was the same were used, extractive distillation was performed in Example 1, and usual distillation was performed in Example 2. The results are set forth in Table 3. Example 1 is Comparative Example, and Example 2 is Example.

[Table 3]

| | | Example 1 | Example 2 | |
|---|---|---|---|---|
| | | Usual distillation | Extractive distillation | Solvent collection distillation |
| Extraction solvent | - | - | R32 | |
| VdF supply amount | kmol/h | 2.70 | 2.70 | - |
| R23 supply amount | kmol/h | 0.30 | 0.30 | - |
| Solvent supply amount | kmol/h | - | | - |
| Column pressure | MPaG | 0.90 | 0.90 | 0.70 |
| Distillation column | Number of plates | 80 | 80 | 30 |

(continued)

| | | Example 1 | Example 2 | |
|---|---|---|---|---|
| | | Usual distillation | Extractive distillation | Solvent collection distillation |
| Supply plate | Plate | 40 | - | 15 |
| Solvent supply plate | Plate | - | 20 | - |
| Gas supply plate | Plate | - | 75 | - |
| Reflux ratio | - | 15 | 25 | 6.5 |
| Reboiler load | kcal/h | 23000 | 23000 | 57000 |
| Distillation temperature | °C | -31.71 | -29.75 | -47.84 |
| VdF distillation amount | kmol/h | 0.43 | 0.62 | 2.08 |
| R23 distillation amount | kmol/h | 0.25 | 0.00 | 0.30 |
| Solvent distillation amount | kmol/h | - | 0.00 | 0.00 |
| Distillate VdF purity | mol% | 62.91 | 99.94 | - |
| Bottom temperature | °C | -29.61 | -7.06 | -0.47 |
| VdF bottom amount | kmol/h | 2.27 | 2.08 | 0.00 |
| R23 bottom amount | kmol/h | 0.05 | 0.30 | 0.00 |
| Solvent bottom amount | kmol/h | - | 13.22 | 13.22 |
| Bottom VdF purity | mol% | 98.01 | - | - |

As set forth in Table 3, the purity of VdF was 98.01% by mol in Example 1 while the purity of VdF was 99.94% by mol in Example 2.

Conventionally, in the case of an extraction solvent having a high boiling point of 40°C to 250°C, distillation for collecting the extraction solvent included in a bottom after extractive distillation has tended to require a large amount of energy. In contrast, in Example 2, the boiling point of an extraction solvent was -60 to 0°C, and an extraction solvent was able to be collected at a lower energy than in a conventional case.

In the distillate in the extractive distillation, the contents of R23 and R32 were small, and therefore expressed as 0.00 in Table 3. Specifically, the content of R23 was 563 ppm by mol, and the content of R32 was 16 ppm by mol.

The entire content of the disclosure by Japanese Patent Application No. 2023-030193 filed on February 28, 2023 is incorporated herein by reference. All documents, patent applications, and technical standards described in this specification are herein incorporated by reference to the same extent as if each individual document, patent application, or technical standard was specifically and individually indicated to be incorporated by reference.

**Claims**

1. A method of separating vinylidene fluoride and trifluoromethane, the method comprising:

   a step of mixing a mixture comprising vinylidene fluoride and trifluoromethane with a compound having a boiling point of from -60 to 0°C, to obtain a mixture for extraction; and
   a step of distilling the mixture for extraction, to independently obtain a distillate comprising vinylidene fluoride as a main component, and a bottom product comprising a compound having a boiling point of from -60 to 0°C as a main component and further comprising trifluoromethane.

2. The method of separating vinylidene fluoride and trifluoromethane according to claim 1, wherein an interaction distance $Ra^{R23}$ between trifluoromethane and the compound having a boiling point of from -60 to 0°C, determined from a Hansen solubility parameter value, is less than an interaction distance $Ra^{VdF}$ between vinylidene fluoride and the compound having a boiling point of from -60 to 0°C.

3. The method of separating vinylidene fluoride and trifluoromethane according to claim 2, wherein a ratio of the

9

interaction distance $Ra^{R23}$ to the interaction distance $Ra^{VdF}$ is 0.81 or less.

4. The method of separating vinylidene fluoride and trifluoromethane according to any one of claims 1 to 3, wherein the compound having a boiling point of from -60 to 0°C has a boiling point of from -60 to -10°C.

5. The method of separating vinylidene fluoride and trifluoromethane according to any one of claims 1 to 3, wherein the compound having a boiling point of from -60 to 0°C has a boiling point of from -60 to -30°C.

6. The method of separating vinylidene fluoride and trifluoromethane according to any one of claims 1 to 3, wherein the compound having a boiling point of from -60 to 0°C is a hydrofluorocarbon.

7. The method of separating vinylidene fluoride and trifluoromethane according to any one of claims 1 to 3, wherein the compound having a boiling point of from -60 to 0°C is at least one selected from a group consisting of difluoromethane, chlorofluoromethane, ammonia, isocyanic acid, ethyl azide, azidopropene, formyl fluoride, formaldehyde, and sulfur dioxide.

8. The method of separating vinylidene fluoride and trifluoromethane according to any one of claims 1 to 3, wherein, in the mixture for extraction, a ratio of a molar amount of the compound having a boiling point of from -60 to 0°C to a total molar amount of vinylidene fluoride and trifluoromethane is from 1 to 100.

9. The method of separating vinylidene fluoride and trifluoromethane according to any one of claims 1 to 3, the method further comprising a step of distilling the bottom product, to collect the compound having a boiling point of from -60 to 0°C.

10. The method of separating vinylidene fluoride and trifluoromethane according to claim 9, wherein the collected compound having a boiling point of from -60 to 0°C is reused in the step of obtaining the mixture for extraction.

11. A composition, comprising vinylidene fluoride, trifluoromethane, and a compound having a boiling point of from -60 to 0°C,
wherein a content of the vinylidene fluoride is 99% by mol or more with respect to a total amount of the composition.

12. A method of producing a composition, the method comprising:

a step of mixing a mixture comprising vinylidene fluoride and trifluoromethane with a compound having a boiling point of from -60 to 0°C, to obtain a mixture for extraction; and
a step of distilling the mixture for extraction, to obtain a composition comprising vinylidene fluoride as a main component.

## INTERNATIONAL SEARCH REPORT

| International application No. |
| --- |
| **PCT/JP2024/003332** |

**A. CLASSIFICATION OF SUBJECT MATTER**

*C07C 17/383*(2006.01)i; *C07C 19/08*(2006.01)i; *C07C 21/18*(2006.01)i
FI: C07C17/383; C07C21/18; C07C19/08

According to International Patent Classification (IPC) or to both national classification and IPC

**B. FIELDS SEARCHED**

Minimum documentation searched (classification system followed by classification symbols)

C07C17/383; C07C19/08; C07C21/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2013/151070 A1 (ASAHI GLASS COMPANY, LIMITED) 10 October 2013 (2013-10-10) <br> entire text | 1-12 |
| A | CN 103664507 A (CHANGSHU ZHENFU NEW MATERIALS CO., LTD.) 26 March 2014 (2014-03-26) <br> entire text | 1-12 |
| A | WO 2013/094224 A1 (KUREHA CORPORATION) 27 June 2013 (2013-06-27) <br> entire text | 1-12 |
| A | WO 2014/178352 A1 (ASAHI GLASS COMPANY, LIMITED) 06 November 2014 (2014-11-06) <br> paragraphs [0082], [0083] | 11 |
| A | JP 2019-501862 A (SRF LIMITED) 24 January 2019 (2019-01-24) <br> paragraph [0033] | 11 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 April 2024** | **23 April 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**
Information on patent family members

International application No.

**PCT/JP2024/003332**

| Patent document cited in search report | | | Publication date (day/month/year) | Patent family member(s) | | Publication date (day/month/year) |
|---|---|---|---|---|---|---|
| WO | 2013/151070 | A1 | 10 October 2013 | (Family: none) | | |
| CN | 103664507 | A | 26 March 2014 | (Family: none) | | |
| WO | 2013/094224 | A1 | 27 June 2013 | (Family: none) | | |
| WO | 2014/178352 | A1 | 06 November 2014 | US | 2016/0002518 A1 | |
| JP | 2019-501862 | A | 24 January 2019 | US | 2018/0251416 A1 | |

Form PCT/ISA/210 (patent family annex) (July 2022)

**EP 4 674 831 A1**

**Patent documents cited in the description**

- WO 2015072305 A

- JP 2023030193 A